# EUROPÄISCHE PATENTSCHRIFT

(11) **EP 2 741 730 B1**
(45) Veröffentlichungstag und Bekanntmachung des Hinweises auf die Patenterteilung: **14.10.2015**
(21) Anmeldenummer: 12745475.9
(22) Anmeldetag: 02.08.2012
(51) Int. Cl.: A61K 8/06, A61K 8/37, A61K 8/60, A61Q 19/00, A61K 8/894, A61K 8/90, A61K 9/113, A61K 8/23, A61K 31/713, A61K 33/06

(54) **MULTIPLE EMULSION**
MULTIPLE EMULSION
ÉMULSION MULTIPLE

(30) Priorität: 10.08.2011 DE 102011109868
(43) Veröffentlichungstag der Anmeldung: 18.06.2014
(73) Patentinhaber: Sterna Biologicals GmbH & Co. KG, 35037 Marburg (DE)
(72) Erfinder: RUNKEL, Frank, 35418 Buseck (DE); SCHMIDTS, Thomas, 35394 Giessen (DE)
(74) Vertreter: Patentanwälte Olbricht Buchhold Keulertz
(86) Internationale Anmeldenummer: PCT/EP2012/065200
(87) Internationale Veröffentlichungsnummer: WO 2013/020899

(56) Entgegenhaltungen:
- DE-A1- 10 238 298
- FR-A1- 2 815 637
- FR-A1- 2 848 420
- SCHMIDTS T ET AL: "Development of multiple W/O/W emulsions as dermal carrier system for oligonucleotides: Effect of additives on emulsion stability", INTERNATIONAL JOURNAL OF PHARMACEUTICS, ELSEVIER BV, NL, Bd. 398, Nr. 1-2, 15. Oktober 2010 (2010-10-15), Seiten 107-113, XP027261255, ISSN: 0378-5173 [gefunden am 2010-09-02] in der Anmeldung erwähnt

## Beschreibung

Die Erfindung betrifft eine multiple Emulsion gemäß dem Oberbegriff von Anspruch 1, die Verwendung einer multiplen Emulsion zur Herstellung eines Arzneimittels, eines Medizinprodukts oder eines Kosmetikums gemäß Anspruch 16 und ein Arzneimittel enthaltend eine multiple Emulsion gemäß Anspruch 18 oder 19.

Multiple Wasser-in-Öl-in-Wasser-Emulsionen (W/O/W-Emulsion) sind komplexe Systeme, die eine äußere Wasserphase und eine darin dispergierte Wasser-in-Öl-Emulsion aufweisen, so dass insgesamt sowohl eine Wasser-in-Öl- als auch eine Öl-in-Wasser-Emulsion vorliegt. Aufgrund der damit verbundenen Eigenschaften sind multiple Emulsionen zur Applikation von pharmazeutischen oder kosmetischen Wirkstoffen geeignet, wobei besonders sensitive Wirkstoffe in der inneren wässrigen Phase einer multiplen Emulsion eingeschlossen werden können. Auf diese Weise können die Wirkstoffe einerseits vor äußeren Einflüssen und Degradation geschützt, und andererseits zeitverzögert freigegeben werden.

Im Stand der Technik sind bereits verschiedene multiple Emulsionen beschrieben. Ein grundsätzliches Problem solcher multipler Emulsionen besteht jedoch darin, dass diese wegen der an den Phasengrenzen zwischen der Wasserphase und der Ölphase vorliegenden Grenzflächen häufig instabil sind. In der Folge können im Laufe der Zeit eine stark veränderte Viskosität, eine Trennung oder Inversion der Phasen, oder der Verlust der Multiplizität auftreten.

Die Stabilität einer multiplen Emulsion wird von mehreren Parametern beeinflusst. Schmidts et al. (T. Schmidts, D. Dobler, P. Schlupp, C. Nissing, H. Garn, F. Runkel, International Journal of Pharmaceutics 398 (2010) 107-113) haben beispielsweise den Einfluss der Zusammensetzung einer in der Ölphase dispergierten inneren Wasserphase auf die Stabilität einer multiplen Emulsion beschrieben. Hierbei zeigte sich, dass die Wahl des Emulgators einen besonders großen Einfluss auf die Stabilität hat, aber auch die Natur des Elektrolyten zeigte einen deutlichen Effekt. Bei der Veränderung einer Zusammensetzung einer multiplen Emulsion ist daher zumeist nicht abschätzbar, ob und wenn ja, in welcher Weise die Eigenschaften, wie Stabilität, Viskosität, Tropfengröße ect., einer multiple Emulsion beeinflusst werden. Aus diesem Grund können multiple Emulsionen, deren Bestandteile und Zusammensetzungen für eine bestimmte Anwendung konzipiert wurden, in aller Regel auch nicht durch Abänderung oder Austausch ihrer Komponenten auf andere Anwendungen übertragen werden. Dies gilt insbesondere dann, wenn die wesentlichen Bestandteile, wie Emulgatoren, Elektrolyte und die Hauptbestandteile der verschiedenen Phasen betroffen sind.

Bei multiplen Emulsionen zur Applikation von sensitiven Wirkstoffen ist jedoch die Stabilität der Formulierung besonders bedeutend. Der Wirkstoff kann nämlich nur dann effektiv vor schädlichen äußeren Einflüssen geschützt werden, wenn er in der innersten Phase eingeschlossen ist und diese Phasenlage auch nicht durch Phasentrennung oder-Inversion verändert wird.

Neben der Phasenstabilität der Emulsion, die eine Grundvoraussetzung für den Schutz eines in der inneren Wasserphase W₂ verkapselten Wirkstoffs darstellt, sind allerdings auch weitere Eigenschaften, wie beispielsweise die Diffusionseigenschaften von Bedeutung. So kann ein verkapselter Wirkstoff z.B. auch dann degeneriert werden, wenn er zwar selbst nicht aus der inneren Phase austritt, aber ein "Schadstoff" von außen in diese Phase hineingelangt.

Bei einer topischen Applikation können einige Wirkstoffe bereits durch natürliche Hautbestandteile beschädigt werden. Dies gilt z.B. für Substanzen, die durch enzymatischem Verdau von auf der Haut befindlichen Enzyme degeneriert werden können. Hierzu zählen insbesondere Peptide und Verbindungen auf Nukleinsäurebasis, wie Poly- und Oligonukleotide, die durch ubiquitäre Nukleasen bzw. Peptidasen vorzeitig abgebaut werden können. Der Einsatz solcher Verbindungen zur Behandlung von Krankheiten hat im Laufe der letzten Jahre jedoch zunehmend an Bedeutung gewonnen. Antisense-Oligonukleotide, aber auch Ribozyme, RNAi, siRNA und insbesondere DNAzyme, können beispielsweise dazu verwendet werden, um in einer Zielzelle die Bildung eines Proteins zu verhindern, indem die Translation der mRNA dieses Proteins unterdrückt wird. Unter DNAzymen werden dabei einzelsträngige DNA-Moleküle verstanden, die enzymatische Aktivität aufweisen und im Gegensatz zu Ribozymen nicht natürlich vorkommen. Problematisch ist dabei jedoch, dass die genannten Nukleotid-Wirkstoffe zumeist sehr empfindlich gegenüber Umweltbedingungen sind und ihre Wirkorte häufig nicht ohne die Hilfe von entsprechenden Trägern erreichen, ohne zuvor degradiert zu werden.

Im Stand der Technik ist bislang keine multiple W/O/W-Emulsion bekannt, die eine ausreichende Langzeitstabilität aufweist, um dauerhaft einen wirksamen Schutz eines derart sensitiven Wirkstoffs gewährleisten zu können.

Bei bisher herkömmlichen W/O/W-Emulsionen ist zudem häufig problematisch, dass sie nur schwerlich für eine Herstellung im Großmaßstab geeignet sind. Auch ein Schutz der W/O/W-Emulsion vor mikrobiellem Befall ist häufig nicht gegeben. Sie sind daher wenn überhaupt so nur äußerst bedingt für die Applikation eines der vorgenannten Wirkstoffe geeignet.

Aufgabe der Erfindung ist es daher diese und weitere Nachteile des Standes der Technik zu überwinden und eine multiple Emulsion zur Applikation eines Wirkstoffs zur Verfügung zu stellen. Die multiple Emulsion soll insbesondere eine hohe Stabilität aufweisen, einen effektiven Schutz des Wirkstoffs vor äußeren Einflüssen bieten, und zugleich einen Schutz der Emulsion vor mikrobiellem Befall ermöglichen. Darüber hinaus soll die multiple Emulsion auch im Großmaßstab einfach und kostengünstig herstellbar sein.

Hauptmerkmale der Erfindung sind im kennzeichnenden Teil der Ansprüche 1, 16, 18 und 19 angegeben. Ausgestaltungen sind Gegenstand der jeweiligen Unteransprüche.

Bei einer multiplen Emulsion zur Applikation wenigstens eines pharmazeutischen oder kosmetischen Wirkstoffs, die eine äußere Wasserphase W₁, eine in der äußeren Wasserphase W₁ dispergierte Ölphase O und eine in der Ölphase O dispergierte innere Wasserphase W₂ aufweist, wobei in der inneren Wasserphase W₂ wenigstens ein Elektrolyt ausgewählt aus der Gruppe der Alkali- und Erdalkalihalogenide und -sulfate und wenigstens ein hydrophiler Wirkstoff vorgesehen ist, sieht die Erfindung vor, dass:
- die äußere Wasserphase W₁ einen hydrophilen Emulgator enthält, der ein Polymer von Ethylenoxid und Propylenoxid ist,
- die Ölphase O von Triacylglyceriden gebildet ist und einen lipophilen Emulgator aus der Gruppe der Silikone aufweist und
- der hydrophile Wirkstoff wenigstens ein Oligonukleotid mit einer Sequenz gemäß SEQ ID No: 1 bis SEQ ID No: 148 oder SEQ ID No: 150 ist.

Die erfindungsgemäße multiple Emulsion weist aufgrund ihrer spezifischen Zusammensetzung eine besonders hohe Stabilität auf. Dies gilt sowohl für die Stabilität bei der Herstellung bis hin zum Größmaßstab, als auch im Hinblick auch die Langzeitstabilität.

Ein weiterer Vorteil ist die einfache Zusammensetzung der erfindungsgemäßen multiplen Emulsion, die eine unkomplizierte und kostengünstige Herstellung ausgehend von einer geringen Anzahl verschiedener Komponenten ermöglicht. Hinzu kommt, dass sämtliche Bestandteile der erfindungsgemäßen Emulsion allenfalls ein geringes Irritationspotential besitzen und im Bereich der Anwendung auf der Haut bereits etabliert sind. Außerdem sind alle Bestandteile problemlos in sehr hohen Reinheitsgraden verfügbar.

Darüber hinaus bietet die erfindungsgemäße multiple Emulsion durch ihre besondere Zusammensetzung einen besonders hohen Schutz für in der inneren Wasserphase W₂ eingeschlossene sensitive Wirkstoffe.

Durch die spezifische Kombination der erfindungsgemäß vorgesehenen Inhaltsstoffe, insbesondere durch die Kombination der vorgesehenen Emulgatoren zusammen mit einer Ölphase aus Triacylglyceriden, kann eine multiple W/O/W-Emulsion bereitgestellt werden, die in vergleichsweise hohe Toleranz gegenüber verschiedenen weiteren Zusatzstoffen aufweist. Dies schafft Handlungsspielraum für gegebenenfalls gewünschte Modifizierungen und/oder Feinabstimmungen. Von besonders großem Vorteil in dieser Hinsicht ist, dass verschiedene Konservierungsmittel zum Schutz vor mikrobiellem Befall zugesetzt werden können, ohne dass die Stabilität der multiplen Emulsion beeinträchtigt wird.

In einer wichtigen Ausführungsform der Erfindung ist der hydrophile Emulgator ein Poloxamer. Poloxamere sind Blockpolymere von Ethylenoxid und Propylenoxid. Ganz besonders bevorzugt ist der Einsatz von Poloxamer 407 als hydrophilen Emulgator.

Eine weitere wichtige Ausgestaltung der Erfindung sieht vor, dass der lipophile Emulgator ein lineares Dimethicon mit Polyether- und Alkylgruppen ist. Besonders bevorzugt sind Dimethicone, die Polyethergruppen, wie Polyethylenglykol- (PEG) und/oder Polypropylenglykolgruppen (PPG), und Alkylgruppen aufweisen, sowie lineare Dimethicone. Ganz besonders gute Ergebnisse werden erzielt, wenn Cetyl PEG/PPG-10/1 Dimethicone (Handelsname: Degussa ABIL® 90 EM) der lipophile Emulgator ist. Durch den Einsatz der genannten Dimethicone als lipophilen Emulgator kann zum einen die Tröpfchengröße der primären w/o Emulsion gut eingestellt werden und die Stabilität der Emulsion erheblich verbessert werden. Zum anderen kann mit dem Einsatz der genannten Dimethicone als lipophilen Emulgator auch die Tröpfchengröße der multiplen w/o/w Emulsion über einen weiten Bereich gut eingestellt werden und die Stabilität der Emulsion. Weiterhin hat die Tröpfchengröße solcher Emulsionen keinen Einfluss mehr auf die Langzeitstabilität. Auch kann eine größere Toleranz gegenüber erforderlichen Zusatzstoffen erreicht werden.

Des Weiteren kann es von Vorteil sein, wenn die erfindungsgemäß vorgesehenen Triacylglyceride Ester gesättigter Fettsäuren, bevorzugt Ester gesättigter Fettsäuren mittlerer Kettenlänge sind. Darunter haben sich Ester von Fettsäuren mit 4 bis 20 Kohlenstoffatomen und vor allem Ester von Fettsäuren mit 6 bis 14 Kohlenstoffatomen als vorteilhaft erwiesen.

Ganz besonders stabile W/O/W-Emulsionen können mit Triglyceriden, die eine der folgenden Kettenlängenmischungen aufweisen, erhalten werden:
1) Kettenlängenmischung (Gaschromatografie):
   C-Kettenlängenverteilung C 10 20,0-50,0 %
   C- Kettenlängenverteilung C 12 <=3,0 %
   C- Kettenlängenverteilung C 14 <=1,0 %
   C- Kettenlängenverteilung C 6 <=2,0 %
   C- Kettenlängenverteilung C 8 50,0-80,0 %
2) Kettenlängenmischung (Gaschromatografie):
   Capronsäure C6 <= 2.0 % 0.1 %
   Caprylsäure C8 50.0 bis 80.0 % 55.7 %
   Caprinsäure C10 20.0 bis 50.0 % 43.3 %
   Laurinsäure C12 <= 3.0 % 0.6 %
   Myristinsäure C14 <= 1.0 % 0.0 %

In einer bevorzugten Ausführungsform der Erfindung ist der Elektrolyt NaCl, MgSO₄ oder ein Gemisch aus NaCl und MgSO₄.

Eine andere Ausgestaltung der Erfindung sieht vor, dass der hydrophile Wirkstoff MgSO₄ ist. MgSO₄ wirkt sich vorteilhaft auf das Hautbild aus. Eine erfindungsgemäße multiple Emulsion mit diesem Wirkstoff ist daher beispielsweise zur unterstützenden Hautpflege und Behandlung bei Neurodermitis geeignet.

Darüber hinaus sieht die Erfindung vor, dass der hydrophile Wirkstoff ein Oligonukleotid ist. Bei einem solchen Oligonukleotid handelt es sich um ein DNAzym, nämlich das 10-23 DNAzym, das als katalytische Domäne die Sequenz SEQ ID No. 149 ggctagctacaacga enthält. Dabei ist die katalytische Domäne von einer rechten und einer linken Substratbindungsdomäne flankiert. Diese ist gegen die mRNA-Sequenz eines Faktors gerichtet, der an einem der Signaltransduktionswege chronisch entzündlicher Krankheiten beteiligt ist. Vorstellbar ist beispielsweise, aber nicht erschöpfend, dass die rechte und die linke Substratbindungsdomäne an eine mRNA binden können, die für einen der folgenden Faktoren codiert: Src-Kinase, Tec-Kinase, Rlk, Itk, RIBP (Rlk/Itk-binding protein), PLCg (Phospholipase Cy1), MAP-Kinase, ERK, JNK, P38, MKK, Rac2, GADD45, SOCS, CIS, JAK, NF-AT interacting protein.

DNAzyme, die als Wirkstoffe in der erfindungsgemäßen multiplen Emulsion verwendet werden, umfassen insofern die allgemeine Sequenz SEQ ID. NO 150: nnnnnnnnnggctagctacaacgannnnnnnnn. Dabei ist die rechte und die linke

Substratbindungsdomäne in den bevorzugten Ausführungsbeispielen jeweils mindestens neuen Basen lang. Selbstverständlich sind jedoch auch kürzere oder längere Sequenzen vorstellbar. Besonders bevorzugt sind dabei Substratbindungsdomänen, die die mRNA der Proteine GATA-3 und T-bet binden. Eine Inhibition der Translation dieser Proteine führt zu einer Unterdrückung der Antwort von T2-Zellen und suprimiert insofern inflammatorische Erkrankungen.

Der hydrophile Wirkstoff ist ein Oligonukleotid mit einer Sequenz gemäß einer der Sequenzen SEQ ID No: 1 bis SEQ ID No: 148 oder der oben dargestellten SEQ ID No: 150. Denkbar ist selbstverständlich auch eine die gleichzeitige Verwendung mehrerer der darggestellten Oligonukleotide. Die katalytische Domäne der DNAzyme ist dabei in Kleinbuchstaben dargestellt, die Substratbindedomänen in Großbuchstaben. Die Sequenzen SEQ ID No. 1 bis SEQ ID No. 71 binden die mRNA von GATA-3. Die Sequenzen SEQ ID No. 72 bis SEQ ID No. 148 binden die mRNA von T-bet.

Ganz besonders bevorzugt wird als hydrophiler Wirkstoff ein Oligonukleotid mit einer Sequenz SEQ ID No: 40, SEQ ID No: 139 oder SEQ ID No: 140 verwendet.

Um einen Schutz der Emulsion vor mikrobiellem Befall gewährleisteten zu können, kann vorgesehen sein, dass die multiple Emulsion ein oder mehrere Konservierungsmittel enthält. Bevorzugte Konservierungsmittel sind Kaliumsorbat, Kaliumsorbat mit Zitronensäure, Sorbinsäure, Benzoesäure, Polyole, wie bevorzugt Pentylenglycol, Butylenglycol und/oder Propylenglycol, Parbene, wie bevorzugt Methylparaben und/oder Propylparaben, sowie Phenoxyethanol. Das Konservierungsmittel ist bevorzugt in der äußeren Wasserphase W₁ und/oder in der inneren Wasserphase W₂ vorgesehen, vorzugsweise in einem Anteil von >0 bis 20 Gew.-% bezogen auf das Wasser.

Eine wichtige Ausführungsform sieht außerdem vor, dass die Tropfengröße der dispergierten Ölphase 1 µm bis 100 µm, bevorzugt 5 µm bis 60 µm und besonders bevorzugt 10 µm bis 30 µm beträgt.

Je nach Anwendung kann es vorteilhaft sein, wenn in der Ölphase ein gesättigter Kohlenwasserstoff enthalten ist. Der bevorzugte gesättigte Kohlenwasserstoff ist Paraffin, insbesondere dickflüssiges Paraffin.

Besonders vorteilhafte Emulsionen können erhalten werden, wenn der hydrophile Emulgator in der äußeren Wasserphase W₁ in einem Anteil von 0,1 bis 5 Gew.-%, bevorzugt 0,3 bis 3 Gew.-%, besonders bevorzugt 0,5 bis 1,5 Gew.-% und ganz besonders bevorzugt in einem Anteil von 0,8 Gew.-% vorliegt.

Außerdem ist die Stabilität der multiplen Emulsion besonders hoch, wenn der lipophile Emulgator bezogen auf eine W/O-Primäremulsion bestehend aus der Ölphase O und der inneren Wasserphase W₂ in einem Anteil von 0,1 bis 6 Gew.-% vorliegt, bevorzugt 0,5 bis 4 Gew.-%, besonders bevorzugt 1 bis 3 Gew.-% und ganz besonders bevorzugt in einem Anteil von 3 Gew.-% vorliegt.

Im Hinblick auf die Triglyceride ist es von Vorteil, wenn die Triglyceride bezogen auf eine W/O-Primäremulsion bestehend aus der Ölphase O und der inneren Wasserphase W₂ in einem Anteil von 5 bis 30 Gew.-%, bevorzugt 10 bis 25 Gew.-%, besonders bevorzugt 15 bis 20 Gew.-% und ganz besonders bevorzugt in einem Anteil von 17 Gew.-% vorliegen.

Der Elektrolyt liegt in einer bevorzugten Ausführungsform bezogen auf eine W/O-Primäremulsion bestehend aus der Ölphase O und der inneren Wasserphase W₂ in einem Anteil von 0,1 bis 3 Gew.-%, besonders bevorzugt 0,3 bis 1,5 Gew.-% und ganz besonders bevorzugt in einem Anteil von 0.7 Gew.-% vor. Dabei kann es sich günstig auswirken, wenn die Elektrolytmenge auf die Wirkstoffmenge abgestimmt wird.

Der hydrophile Wirkstoff liegt bezogen auf die Gesamtmasse der multiplen Emulsion in einem Anteil von 0,01 bis 10 Gew.-%, besonders bevorzugt 0,1 bis 5 und ganz besonders bevorzugt in einem Anteil von 0,4 Gew.-% vor.

In diesem Sinne ist eine erfindungsgemäße multiple Emulsion bevorzugt bei welcher:
- der hydrophile Emulgator in der äußeren Wasserphase W₁ einen Anteil von 0,1 bis 5 Gew.-% aufweist, und
- der lipophile Emulgator bezogen auf eine W/O-Primäremulsion bestehend aus der Ölphase O und der inneren Wasserphase W₂ in einem Anteil von 0,1 bis 6 Gew.-% vorliegt, und
- die Triglyceride bezogen auf die W/O-Primäremulsion in einem Anteil von 5 bis 30 Gew.-% vorliegen, und
- der Elektrolyt bezogen auf eine W/O-Primäremulsion bestehend aus der Ölphase O und der inneren Wasserphase W₂ in einem Anteil von 0,1 bis 3 Gew.-% vorliegt, und
- der hydrophile Wirkstoff bezogen auf die Gesamtmasse der multiplen Emulsion in einem Anteil von 0,01 bis 10 Gew.-% vorliegt, und
- in der äußeren Wasserphase W₁ und/oder der inneren Wasserphase W₂ ein Konservierungsmittel in einem Anteil von 0 bis 20 Gew.-% vorgesehen ist.

Dabei wird vor allem mit solchen erfindungsgemäßen multiplen Emulsionen eine hohe Stabilität und eine effektiver Schutz des Wirkstoffs erreicht, bei denen
bezogen auf eine Primäremulsion W/O
3 Gew.-% lipophiler Emulgator, insbesondere Abil EM 90;
17 Gew.-% Öl, insbesondere mittelkettige Triglyceride;
0,7 Gew.-% Elektrolyt, insbesondere MgSO₄* 7H₂O;
Ad 100 Gew.-% Wasser
vorliegen, wobei bezogen auf die multiple Emulsion
80 Gew.-% W/O Emulsion;
0,8 Gew.-% hydrophiler Emulgator, insbesondere Poloxamer
Ad 100 Gew.-% Wasser enthält
und wobei die fertige multiple W/O/W-Emulsion 0,4 Gew.-% hydrophiler Wirkstoff, insbesondere ein Natriumsalz eines der oben genannten DNAzyme; enthält.

Darüber hinaus sieht die Erfindung die Verwendung einer erfindungsgemäßen multiplen Emulsion zur Herstellung eines Arzneimittels, eines Medizinprodukts oder eines Kosmetikums vor. Hierbei ist es besonders vorteilhaft, wenn die multiple Emulsion als Wirkstoffträger verwendet wird. Dabei kann es sich um einen pharmazeutischen oder kosmetischen Wirkstoff handeln. Auf diese Weise können Arzneimittel bereitgestellt werden, die dazu geeignet sind, hochsensible und hochspezifische Wirkstoffe z.B. durch topische Applikation oder in einer anderen zweckmäßige Form zu verabreichen. Die so gestalteten Arzneimittel sind besonders gut zur Behandlung von Krankheiten geeignet, die auf Fehlfunktionen ganz spezieller molekularbiologischer Faktoren, beispielsweise GATA-3 oder einem der anderen oben genannten Faktoren, zurückzuführen sind.

In einem weiteren Aspekt betrifft die Erfindung daher auch ein Arzneimittel enthaltend eine erfindungsgemäße multiple Emulsion zur topischen Anwendung oder zur Applikation des Wirkstoffs über Schleimhäute oder zur intraperitonealen Applikation des Wirkstoffs, wobei es hierbei ebenfalls besonders vorteilhaft ist, wenn die multiple Emulsion Wirkstoffträger ist. In einer bevorzugten Ausführungsform handelt es sich um ein Arzneimittel zur Behandlung einer inflammatorischen Erkrankung.

Weitere Merkmale, Einzelheiten und Vorteile der Erfindung ergeben sich aus dem Wortlaut der Ansprüche sowie aus der folgenden Beschreibung von Ausführungsbeispielen und der Figuren.

Es zeigen die folgenden Figuren und Sequenzen:
SEQ ID NO: 1 bis SEQ ID NO: 148 Ausführungsbeispiele für 10-23 DNAzym, die an GATA-3 bzw. T-bet binden;
SEQ ID NO: 149 die katalytische Domäne von 10-23 DNAzym;
SEQ ID NO: 150 DNAzym umfassend die katalytische Domäne 10-23 DNAzym;
Fig. 1 Messdaten zur Verifizierung der Schutzfunktion einer erfindungsgemäßen Emulsion;
Fig. 2 die innere Struktur einer multiplen Vergleichsemulsion; und
Fig. 3 die innere Struktur einer multiplen Emulsion aus der Basisrezeptur WOW_1 (Zusammensetzung s. Ausführungsbeispiele Punkt 2)).

### Ausführungsbeispiele:

In den nachfolgend dargestellten Ausführungsbeispielen wurden soweit nicht anderweitig angegeben folgende Inhaltsstoffe verwendet:

| Inhaltstoffe | Herstellername | Hersteller |
|---|---|---|
| Innere Wasserphase W₂: | | |
| Destilliertes Wasser | | Fagron |
| MgSO₄·7H₂O | | Biospring |
| DNAzyme Na-Salz | Magnesiumsulfat heptahydrat | Fagron |
| Kaliumsorbat | | Fagron |
| Zitronensäure | | |
| | | |
| Ölphase O: | | Fagron |
| Mittelkettige Triglyceride | | Evonik |
| Cetyl PEG/PPG-10/1 | Abil EM 90 | |
| Dimethicone | | |
| | | |
| Äußere Wasserphase W₁: | | |
| Destilliertes Wasser | | Fagron |
| Kaliumsorbat | | Fagron |
| Zitronensäure | | Fagron |
| Poloxamer 407 | | |

### 1) Allgemeines Herstellungsverfahren

Die nachfolgend beschriebenen Emulsionen wurden nach folgendem allgemeinen Herstellungsverfahren hergestellt:

Zunächst werden drei Phasen A, B und C hergestellt. Zur Herstellung der Phase A werden die für die innere Wasserphase W₂ der Emulsion vorgesehen Bestandteile gemischt, nämlich beispielsweise destilliertes Wasser, Magnesiumsulfat heptahydrat, DNAzym, Kaliumsorbat und Zitronensäure in den in den nachfolgenden Listen angegebenen Mengen. Zur Herstellung der Phase B werden die für die Ölphase O der Emulsion vorgesehen Bestandteile gemischt, nämlich beispielsweise mittelkettige Triglyceride und Abil ® EM 90 in den in den nachfolgenden Listen angegebenen Mengen. Zur Herstellung der Phase C werden die für die äußere Wasserphase C vorgesehenen Bestandteile gemischt, nämlich beispielsweise destilliertes Wasser, Kaliumsorbat, Zitronensäure und Poloxamer 407 in den in den nachfolgenden Listen angegebenen Mengen.

Danach werden Phase A und Phase B einzeln auf ca. 60-70°C erwärmt. Anschließend wird Phase A in Phase B gegeben und ca. 3 min homogenisiert (Homogenisator: ca. 11500 rpm), wobei sich die W/O-Primäremulsion bildet. Die W/O-Primäremulsion wird unter Rühren auf Raumtemperatur abgekühlt (Rührer: ca. 500 rpm) und anschließend mit der Phase C versetzt. Nach der Zugabe der Phase C wird solange gerührt (Rührer: ca. 1200 rpm) und/oder homogenisiert, bis eine gleichmäßige Emulsion entstanden ist (Dauer ca. 10 min).

Durch das genannte Herstellungsverfahren sind beispielsweise multiple Emulsionen hergestellt, bei denen:
- der hydrophile Emulgator in der äußeren Wasserphase W₁ einen Anteil von 0,1 bis 5 Gew.-% aufweist, und
- der lipophile Emulgator bezogen auf eine W/O-Primäremulsion bestehend aus der Ölphase O und der inneren Wasserphase W₂ in einem Anteil von 0,1 bis 6 Gew.-% vorliegt, und
- die Triglyceride bezogen auf die W/O-Primäremulsion in einem Anteil von 5 bis 30 Gew.-% vorliegen, und
- der Elektrolyt bezogen auf eine W/O-Primäremulsion bestehend aus der Ölphase O und der inneren Wasserphase W₂ in einem Anteil von 0,1 bis 3 Gew.-% vorliegt, und
- der hydrophile Wirkstoff bezogen auf die Gesamtmasse der multiplen Emulsion in einem Anteil von 0,01 bis 10 Gew.-% vorliegt, und
- in der äußeren Wasserphase W₁ und/oder der inneren Wasserphase W₂ ein Konservierungsmittel in einem Anteil von 0 bis 20 Gew.-% vorgesehen ist.

Insbesondere wurden mit Hilfe des Herstellungsverfahrens Rezepturen hergestellt, bei denen:
bezogen auf eine Primäremulsion W/O
   3 Gew.-% lipophiler Emulgator, insbesondere Abil EM 90;
   17 Gew.-% Öl, insbesondere mittelkettige Triglyceride;
   0,7 Gew.-% Elektrolyt, insbesondere MgSO₄* 7H₂O;
   Ad 100 Gew.-% Wasser
vorliegen, wobei bezogen auf die multiple Emulsion
   80 Gew.-% W/O Emulsion;
   0,8 Gew.-% hydrophiler Emulgator, insbesondere Poloxamer
   Ad 100 Gew.-% Wasser enthält,
und wobei die fertige multiple W/O/W-Emulsion 0,4 Gew.-% hydrophiler Wirkstoff, insbesondere ein Natriumsalz eines der nachfolgend in Punkt 4) der Ausführungsbeispiele genannten DNAzyme; enthält.

Beispielhaft wird die Herstellung folgender multipler Emulsionen genannt:

### WOW_A1 (100g Maßstab) :

| | | | |
|---|---|---|---|
| A | MgSO₄·7H₂O | 0.56 g | 0,56Gew.-%" |
| | Destilliertes Wasser | 63,307 g | 63,307 Gew.-%^{a} |
| | Kaliumsorbat | 0,089 g | 0,089 Gew.-%^{a} |
| | Zitronensäure | 0,044 g | 0,044 Gew.-%^{a} |
| | | | |
| B | Mittelkettige Triglyceride | 13,6 g | 13,6 Gew.-%^{a} |
| | Cetyl PEG/PPG-10/1 Dimethicone | 2,4 g | 2,4 Gew.-%^{a} |
| | | | |
| C | Destilliertes Wasser | 9,160 g | 19,160 Gew.-%^{a} |
| | Kaliumsorbat | 0,027 g | 0,027 Gew.-%^{a} |
| | Zitronensäure | 0,013 g | 0,013 Gew.-%^{a} |
| | Poloxamer 407 | 0,8 g | 0,8 Gew.-%^{a} |

| | | | |
|---|---|---|---|
| ^{a} Gew.-% bezogen auf die Gesamtmasse der multiplen Emulsion | | | |

### WOW_A2 (12000g Maßstab):

| | | | |
|---|---|---|---|
| A | MgSO₄·7H₂O | 7,2 g | 0,56 Gew.-%^{a} |
| | Destilliertes Wasser | 7596,813 g | 63,307 Gew.-%^{a} |
| | Kaliumsorbat | 10,658 g | 0,089 Gew.-%^{a} |
| | Zitronensäure | 5.329 g | 0,044 Gew.-%^{a} |
| | | | |
| B | Mittelkettige Triglyceride | 1632 g | 13,6 Gew.-%^{a} |
| | Cetyl PEG/PPG-10/1 Dimethicone | 288 g | 2,4 Gew.-%^{a} |
| | | | |
| C | Destilliertes Wasser | 2299,162 g | 19,160 Gew.-%^{a} |
| | Kaliumsorbat | 3,226 g | 0,027 Gew.-%^{a} |
| | Zitronensäure | 1,612 g | 0,013 Gew.-%^{a} |
| | Poloxamer 407 | 96 g | 0,8 Gew.-%^{a} |

| | | | |
|---|---|---|---|
| ^{a} Gew.-% bezogen auf die Gesamtmasse der multiplen Emulsion | | | |

### WOW_A3 (100g Maßstab) :

| | | | |
|---|---|---|---|
| A | MgSO₄·7H₂O | 0,56 g | 0,56 Gew.-%^{a} |
| | Destilliertes Wasser | 62,907 g | 62,907 Gew.-%^{a} |
| | Na-Salz von DNAzyme | 0,4 g | 0,4 Gew.-%^{a} |
| | Kaliumsorbat | 0,089 g | 0,089 Gew.-%^{a} |
| | Zitronensäure | 0,044 g | 0,044 Gew.-%^{a} |
| | | | |
| B | Mittelkettige Triglyceride | 13,6 g | 13,6 Gew.-%^{a} |
| | Cetyl PEG/PPG-10/1 Dimethicone | 2,4 g | 2,4 Gew.-%^{a} |
| | | | |
| C | Destilliertes Wasser | 19,160 g | 19,160 Gew.-%^{a} |
| | Kaliumsorbat | 0,027 g | 0,027 Gew.-%^{a} |
| | Zitronensäure | 0,013 g | 0,013 Gew.-%^{a} |
| | Poloxamer 407 | 0,8 g | 0,8 Gew.-%^{a} |

| | | | |
|---|---|---|---|
| ^{a} Gew.-% bezogen auf die Gesamtmasse der multiplen Emulsion | | | |

### 2) Basisrezeptur einer erfindungsgemäßen multiplen Emulsion WOW_1:

**Tab. 1. Zusammensetzung der W/O/W Emulsion WOW_1**

| | Inhaltsstoffe | [w/w]/% |
|---|---|---|
| WO: | | |
| | Abil EM 90 (lipophiler Emulgator) | 3 |
| | Mittelkettige Triglyceride (Öl) | 17 |
| | | |
| | MgSO4*7H2O (Elektrolyt und/oder Wirkstoff) | 0,7 |
| | H2O | 79,3 |
| WOW: | | |
| | WO | 80 |
| | Poloxamer 407 (hydrophiler Emulgator) | 0,8 |
| | H2O | 19,2 |

Bei der dargestellten Ausführungsform der Erfindung ist sowohl der Elektrolyt als auch der hydrophile Wirkstoff MgSO₄. Als lipophiler Emulgator wird ein Dimethicon, namentlich Abil EM 90, verwendet. Triacyglyceride, nämlich mittelkettige Triglyceride, bilden das Öl. Der hydrophile Emulgator ist ein Polymer von Ethylenoxid und Propylenoxid, namentlich Poloxamer 407.

Die Basisrezeptur WOW_1 wird nach dem oben genannten allgemeinen Herstellungsverfahren hergestellt.

Die Tropfengrößen der dispergierten Ölphase der multiplen Emulsion WOW_1 liegen bei 30 µm, die Viskosität beträgt 1,2 Pa s.

Führt man Langzeitstabilitätsmessungen in einem Zeitraum von beispielsweise 9 Monaten durch, bleibt in diesem Zeitraum die Emulsion stabil. Sie zeigt keine signifikante Veränderung der Tropfengröße und der Viskosität.

### 3) Einfluss der Zusammensetzung auf die Stabilität

### 3.1) Einfluss des lipophilen Emulgators auf die Stabilität einer W/O/W-Emulsion

Um den Einfluss des lipophilen Emulgators zu untersuchen, stellt man eine multiple Emulsion WOW_1 gemäß Ausführungsbeispiel 1) her, wobei zusätzlich zu Abil 90 EM Sorbitan monooleate als ein weiterer lipophiler Emulgator zur primären Emulsion zugegeben wird.

Die Veränderung des lipophilen Emulgators führt zu einer Destabilisierung der W/O/W-Emulsion.

### 3.2) Weitere Modifikationen

| Inhaltsstoffe | Tropfengröße | Viskosität | Phasenstabilität |
|---|---|---|---|
| Basisrezeptur | +++ | +++ | +++ |
| M1: | + | - | + |
| WOW 250 | | | |
| | | | |
| W₂: MgSO4 | | | |
| Ölphase: Abil EM 90, Mittelkettige Triglyceride, Decyl Oleate, Lecithin | | | |
| W₁: Poloxamer 407 Konservierungsmittel: Butylenglycol, Glycerin | | | |
| M2: | ++ | ++ | +++ |
| WOW 251 | | | |
| | | | |
| W₂: MgSO4 | | | |
| Ölphase: Abil EM 90, Mittelkettige Triglyceride, Decyl Oleate, Sorbitan Monooleate | | | |
| W₁: Poloxamer 407 Konservierungsmittel: Butylenglycol, Glycerin | | | |
| M3: | + | - | + |
| WOW 253 | | | |
| | | | |
| W₂: MgSO4, NaCl Ölphase: Abil EM 90, Paraffin dickflüssig, Lecithin, Sorbitan Monooleate | | | |
| W₁: Steareth-20 Konservierungsmittel: Benzoesäure | | | |
| M4: | +++ | + | +++ |
| WOW 255 | | | |
| | | | |
| W₂: MgSO4 | | | |
| Ölphase: Abil EM 90, Mittelkettige Triglyceride, | | | |
| W₁: Steareth-20 Konservierungsmittel: Butylenglycol | | | |
| M5 | + | + | +++ |
| WOW 256 | | | |
| | | | |
| W₂: MgSO4 | | | |
| Ölphase: Abil EM 90, Paraffin dickflüssig, | | | |
| W₁: Poloxamer 407 Konservierungsmittel: Butylenglycol | | | |
| M6: | + | + | +++ |
| WOW 257 | | | |
| | | | |
| W₂: MgSO4 | | | |
| Ölphase: Abil EM 90, Mittelkettige Triglyceride, | | | |
| W₁: Steareth-20 Konservierungsmittel: Butylenglycol, Glycerin | | | |
| M7: | ++ | ++ | +++ |
| WOW 260 | | | |
| | | | |
| W₂: MgSO4 | | | |
| Ölphase: Abil EM 90, Mittelkettige Triglyceride, Decyl Oleate (nicht Ph.Eur.) | | | |
| W₁: Poloxamer 407 Konservierungsmitte: Kaliumsorbat, Glycerin | | | |

| | | | |
|---|---|---|---|
| Bewertungsschlüssel: +++ sehr gut ++ gut + befriedigend - schlecht | | | |

Wie die Versuchsergebnisse zeigen, liefern nur die Emulsionen M1 bis M7, gute Resultate. Dies zeigt, dass die erfindungsgemäße Emulsion eine hohe Toleranz gegenüber verschiedenen Zusatzstoffen zeigt.

### 4) Schutzwirkung der multiplen Emulsion

Zur Verifizierung der Schutzwirkung der multiplen Emulsion versetzt man verschiedene Formulierungen, die ein DNAzym als ein Beispiel für einen sensitiven Wirkstoff auf Nukleotidbasis enthalten, mit einer Nuklease-Lösung.

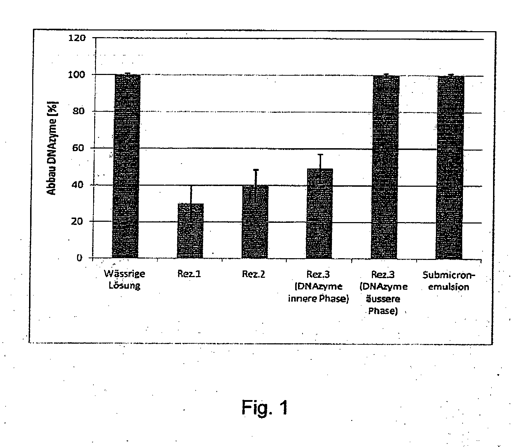

Die Versuchsergebnisse sind als Diagramm in Fig. 1 dargestellt.

### Versuchsdurchführung:

20 mg der jeweiligen Formulierungen werden mit 1 ml einer DNAse I- Lösung (hauteigene DNAsen) vermischt. Nach der Inkubationszeit von 1 min wird das Gemisch für 10 min in einen Thermomixer bei einer Temperatur von 99°C leicht geschüttelt, um die Aktivität der DNAse zu stoppen. Zum Aufbruch der Emulsion wird der Ansatz in dein Ultraschallbad bei 50°C 10 min inkubiert. Ansatz wird durch einen 0,45 µm Spritzenfilter filtriert und Intakte Wirkstoffmenge per HPLC Analyse ermittelt.

Alle Formulierungen enthalten 0,4% eines DNAzyms (Na-Salz). Die Formulierungen Rez. 1, 2 (Spalte 2 und 3) enthalten das DNAzym in der inneren Wasserphase W₂. Der Rez. 3 wurde DNAzym zum Vergleich einmal in der inneren Wasserphase W₂ (Spalte 4) und einmal in der äußeren Wasserphase W₁ zugesetzt (Spalte 5) zugesetzt. Spalte 1 zeigt das Versuchsergebnis, das mit einer einfachen wässrigen Lösung des DNAzyms enthalten wurde und Spalte 6 zeigt das Versuchsergebnis, das mit einer Submicronemulsion erhalten wurde.

Als DNAzym wird jeweils ein DNAzym, welches eine der folgenden Sequenzen aufweist verwendet:
SEQ ID. NO 150: nnnnnnnnnggctagctacaacgannnnnnnnn. Dabei ist die rechte und die linke Substratbindungsdomäne in den bevorzugten Ausführungsbeispielen jeweils mindestens neuen Basen lang.
SEQ ID No: 1
   hgd1 5'-TCGGTCAGAggctagctacaacgaTGCGTTGCT-3'
SEQ ID No: 2
   hgd2 5'-GGCGTACGAggctagctacaacgaCTGCTCGGT-3'
SEQ ID No: 3
   hgd3 5'-GGCGGCGTAggctagctacaacgaGACCTGCTC-3'
SEQ ID No: 4
   hgd4 5'-CTCGGGTCAggctagctacaacgaCTGGGTAGC-3'
SEQ ID No: 5
   hgd5 5'-TCCTCTGCAggctagctacaacgaCGGGGTCCT-3'
SEQ ID No: 6
   hgd6 5'-ACTCTGCAAggctagctacaacgaTCTGCGAGC-3'
SEQ ID No: 7
   hgd7 5'-GGGCGACGAggctagctacaacgaTCTGCAATT-3'
SEQ ID No: 8
   hgd8 5'-AAGGGGCGAggctagctacaacgaGACTCTGCA-3'
SEQ ID No: 9 ,
   hgd9 5'-AAAACGGGAggctagctacaacgaCAGGTTGTA-3'
SEQ ID No: 10
   hgd10 5'-AGAATAAAAggctagctacaacgaGGGACCAGG-3'
SEQ ID No: 11
   hgd11 5'-ATGGCAGAAggctagctacaacgaAAAACGGGA-3'
SEQ ID No: 12
   hgd12 5'-AACTGGGTAggctagctacaacgaGGCAGAATA-3'
SEQ ID No: 13
   hgd13 5'-ATCCAAAAAggctagctacaacgaTGGGTATGG-3'
SEQ ID No: 14
   hgd14 5'-AGGGGAAGAggctagctacaacgaAAAAATCCA-3'
SEQ ID No: 15
   hgd15 5'-TTTTAAAAAggctagctacaacgaTATCTTGGA-3'
SEQ ID No: 16
   hgd16 5'-GTGGGGGGAggctagctacaacgaGGGAAGGCT-3'
SEQ ID No: 17
   hgd17 5'-GTTGAATGAggctagctacaacgaTTGCTTTCG-3'
SEQ IN No: 18
   hgd18 5'-GTCGTTGAAggctagctacaacgaGATTTGCTT-3'
SEQ ID No: 19
   hgd19 5'-GGCCCGGAAggctagctacaacgaCCGCGCGCG-3'
SEQ ID No: 20
   hgd20 5'-TCACCTCCAggctagctacaacgaGGCCTCGGC-3'
SEQ ID No: 21
   hgd21 5'-CCGCCGTCAggctagctacaacgaCTCCATGGC-3'
SEQ ID No: 22
   hgd22 5'-GGTGGCTCAggctagctacaacgaCCAGCGCGG-3'
SEQ ID No: 23
   hgd23 5'-CGTTGAGCAggctagctacaacgaGGCGGGGTG-3'
SEQ ID No: 24
   hgd24 5'-CCGCGTCCAggctagctacaacgaGTAGGAGTG-3'
SEQ ID No: 25
   hgd25 5'-CAGCGGGTAggctagctacaacgaTGCGCCGCG-3'
SEQ ID No: 26
   hgd26 5'-GCACATCCAggctagctacaacgaCTCCTCCGG-3'
SEQ ID No: 27
   hgd27 5'-AAAAGCACAggctagctacaacgaCCACCTCCT-3'
SEQ ID No: 28
   hgd28 5'-TAAAAAGCAggctagctacaacgaATCCACCTC-3'
SEQ ID No:29
   hgd29 5'-GACCGTCGAggctagctacaacgaGTTAAAAAG-3'
SEQ ID No: 30
   hgd30 5'-TTGCCTTGAggctagctacäacgäCGTCGATGT-3'
SEQ ID No: 31
   hgd31 5'-AGGGCGGGAggctagctacaacgaGTGGTTGCC-3'
SEQ ID No: 32
   hgd32 5'-TGGCCCTGAggctagctacaacgaCGAGTTTCC-3'
SEQ ID No: 33
   hgd33 5'-ACCTCTGCAggctagctacaacgaCGTGGCCCT-3'
SEQ ID No: 34
   hgd34 5'-CGGAGGGTAggctagctacaacgaCTCTGCACC-3'
SEQ ID No: 35
   hgd35 5'-GGCGGCACAggctagctacaacgaCTGGCTCCC-3'
SEQ ID No: 36
   hgd36 5'-CGGGCGGCAggctagctacaacgaACCTGGCTC-3'
SEQ ID No: 37
   hgd37 5'-AGGGATCCAggctagctacaacgaGAAGCAGAG-3'
SEQ ID No: 38
   hgd38 5'-GGGTAGGGAggctagctacaacgaCCATGAAGC-3'
SEQ ID No: 39
   hgd39 5'-GGGCTGAGAggctagctacaacgaTCCAGGGGG-3'
SEQ ID No: 40
   hgd40 5'-GTGGATGGAggctagctacaacgaGTCTTGGAG-3'
SEQ ID No: 41
   hgd41 5'-CGTGGTGGAggctagctacaacgaGGACGTCTT-3'
SEQ ID No: 42
   hgd42 5'-GGGGGTAGAggctagctacaacgaGGAGAGGGG-3'
SEQ ID No: 43
   hgd43 5'-GGAGGAGGAggctagctacaacgaGAGGCCGGG-3'
SEQ ID No: 44
   hgd44 5'-GCCCCCCGAggctagctacaacgaAAGGAGGAG-3'
SEQ ID No: 45
   hgd45 5'-CCGGGGAGAggctagctacaacgaGTCCTTCGG-3'
SEQ ID No: 46
   hgd46 5'-GGACAGCGAggctagctacaacgaGGGTCCGGG-3'
SEQ ID No: 47
   hgd47 5'-TGGGGTGGAggctagctacaacgaAGCGATGGG-3'
SEQ ID No: 48
   hgd48 5'-CTTGAGGCAggctagctacaacgaTCTTTCTCG-3'
SEQ ID No: 49
   hgd49 5'-CACCTGGTAggctagctacaacgaTTGAGGCAC-3'
SEQ ID No: 50
   hgd50 5'-GCAGGGGCAggctagctacaacgaCTGGTACTT-3'
SEQ ID No: 51
   hgd51 5'-CCAGCTTCAggctagctacaacgaGCTGTCGGG-3'
SEQ ID No: 52
   hgd52 5'-GTGGGACGAggctagctacaacgaTCCAGCTTC-3'
SEQ ID No: 53
   hgd53 5'-GGAGTGGGAggctagctacaacgaGACTCCAGC-3'
SEQ ID No: 54
   hgd54 5'-ATGCTGCCAggctagctacaacgaGGGAGTGGG-3'
SEQ ID No: 55
   hgd55 5'-GGGCGGTCAggctagctacaacgaGCTGCCACG-3'
SEQ ID No: 56
   hgd56 5'-GAGGCTCCAggctagctacaacgaCCAGGGCGG-3'
SEQ ID No: 57
   hgd57 5'-GTGGGTCGAggctagctacaacgaGAGGAGGCT-3'
SEQ ID No: 58
   hgd58 5'-AGGTGGTGAggctagctacaacgaGGGGTGGTG-3'
SEQ ID No: 59
   hgd59 5'-ACTCGGGCAggctagctacaacgaGTAGGGCGG-3'
SEQ ID No: 60
   hgd60 5'-GGAGCTGTAggctagctacaacgaTCGGGCACG-3'
SEQ ID No: 61
   hgd61 5'-GGACTTGCAggctagctacaacgaCCGANGCCG-3'
SEQ ID No: 62
   hgd62 5'-GGGCCTGGAggctagctacaacgaTTGCATCCG-3'
SEQ ID No: 63
   hgd63 5'-TGTGCTGGAggctagctacaacgaCGGGCCTTG-3'
SEQ ID No: 64
   hgd64 5'-GTTCACACAggctagctacaacgaTCCCTGCCT-3'
SEQ ID No: 65
   hgd65 5'-CAGTTCACAggctagctacaacgaACTCCCTGC-3'
SEQ ID No: 66
   hgd66 5'-CACAGTTCAggctagctacaacgaACACTCCCT-3'
SEQ ID No: 67
   hgd67 5'-GTTGCCCCAggctagctacaacgaAGTTCACAC-3'
SEQ ID No: 68
   hgd68 5'-TCGCCGCCAggctagctacaacgaAGTGGGGTC-3'
SEQ ID No: 69
   hgd69 5'-CCCGTGCCAggctagctacaacgaCTCGCCGCC-3'
SEQ ID No: 70
   hgd70 5'-GGCGTTGCAggctagctacaacgaAGGTAGTGT-3'
SEQ ID No: 71
   td1 5'-TGGCTTCTAggctagctacaacgaGCCCTCGTC-3'
SEQ ID No: 72
   td2 5'-GGGCTCTGAggctagctacaacgaGCCTGGCTT-3'
SEQ ID No: 73
   td3 5'-GGGACCCCAggctagctacaacgaCGGAGCCCG-3'
SEQ ID No: 74
   td4 5'-GGTGGGGGAggctagctacaacgaCCCACCGGA-3'
SEQ ID No: 75
   td5 5'-GGCGGGGGAggctagctacaacgaCCGAGGGCC-3'
SEQ ID No: 76
   td6 5'-GGGCTGGGAggctagctacaacgaGGGCAGGGA-3'
SEQ ID No: 77
   td7 5'-CGTCGAGGAggctagctacaacgaCCGCCCCTC-3'
SEQ ID No: 78
   td8 5'-GGGCTGGCAggctagctacaacgaCTTCCCGTA-3'
SEQ ID No: 79
   td9 5'-CGATGCCCAggctagctacaacgaCCGGGGCGG-3'
SEQ ID No: 80
   td10 5'-GCTCCACGAggctagctacaacgaGCCCATCCG-3'
SEQ ID No: 81
   td11 5'-CCGGCTCCAggctagctacaacgaGATGCCCAT-3'
SEQ ID No: 82
   td12 5'-TCTCCGCAAggctagctacaacgaCCGGCTCCA-3'
SEQ ID No: 83
   td 13 5'-CCGTCAGCAggctagctacaacgaGTCTCCGCA-3'
SEQ ID No: 84
   td14 5'-TCCCCGGCAggctagctacaacgaCGGCTCGGT-3'
SEQ ID No: 85
   td15 5'-CCCCCGCGAggctagctacaacgaGCTCGTCCG-3'
SEQ ID No: 86
   td16 5'-GTAGGGAGAggctagctacaacgaCCCAGGCTG-3'
SEQ ID No: 87
   td17 5'-GGGCGGGCAggctagctäcaacgaCAAGGCGCC-3'
SEQ ID No: 88
   td18 5'-CGGGAAGGAggctagctacaacgaTCGCCCGCG-3'
SEQ ID No: 89
   td19 5'-TAGTCCTCAggctagctacaacgaGCGGCCCCG-3'
SEQ ID No: 90
   td20 5'-TCCCCGACAggctagctacaacgaCTCCAGTCC-3'
SEQ ID No: 91
   td21 5'-TTTCCCCGAggctagctacaacgaACCTCCAGT-3'
SEQ ID No: 92
   td22 5'-TGAGCGCGAggctagctacaacgaCCTCAGTTT-3'
SEQ ID No: 93
   td23 5'-GGACCACAAggctagctacaacgaAGGTGGTTG-3'
SEQ ID No: 94
   td24 5'-CTTGGACCAggctagctacaacgaAACAGGTGG-3'
SEQ ID No: 95
   td25 - 5'-AAACTTGGAggctagctacaacgaCACAACAGG-3'
SEQ ID No: 96
   td26 5'-CTGATTAAAggctagctacaacgaTTGGACCAC-3'
SEQ ID No: 97
   td27 5'-TGGTGCTGAggctagctacaacgaTAAACTTGG-3'
SEQ ID No: 98
   td28 5'-TGATGATCAggctagctacaacgaCTCTGTCTG-3'
SEQ ID No: 99
   td29 5'-TGGTGATGAggctagctacaacgaCATCTCTGT-3'
SEQ ID No: 100
   td30 5'-GCTTGGTGAggctagctacaacgaGATCATCTC-3'
SEQ ID No: 101
   td31 5'-ATGGGAACAggctagctacaacgaCCGCCGTCC-3'
SEQ ID No:102
   td32 5'-GAATGGGAAggctagctacaacgaATCCGCCGT-3'
SEQ ID No: 103
   td33 5'-TGACAGGAAggctagctacaacgaGGGAACATC-3'
SEQ ID No: 104
   td34 5'-AGT'AAATGAggctagctacaacgaAGGAATGGG-3'
SEQ ID No: 105
   td35 5'-CACAGTAAAggctagctacaacgaGACAGGAAT-3'
SEQ ID No: 106
   td36 5'-GCCCGGCCAggctagctacaacgaAGTAAATGA-3'
SEQ ID No: 107
   td37 5'-CCACAAACAggctagctacaacgaCCTGTAGTG-3'
SEQ ID No: 108
   td38 5'-GTCCACAAAggctagctacaacgaATCCTGTAG-3'
SEQ ID No: 109
   td39 5'-CCACGTCCAggctagctacaacgaAAACATCCT-3'
SEQ ID No: 110
   td40 5'-CCAAGACCAggctagctacaacgaGTCCACAAA-3'
SEQ ID No: 111
   td41 5'-CCACCAAGAggctagctacaacgaCACGTCCAC-3'
SEQ ID No: 112
   td42 5'-GCTGGTCCAggctagctacaacgaCAAGACCAC-3'
SEQ ID No: 113
   td43 5'-GCTCTGGTAggctagctacaacgaCGCCAGTGG-3'
SEO ID No: 114
   td44 5'-CTGCACCCAggctagctacaacgaTTGCCGCTC-3'
SEQ ID No: 115
   td45 5'-CACACTGCAggctagctacaacgaCCACTTGCC-3'
SEQ ID No: 116
   td46 5'-CTTTCCACAggctagctacaacgaTGCACCCAC-3'
SEQ ID No: 117
   td47 5'-GCCTTTCCAggctagctacaacgaACTGCACCC-3'
SEQ ID No: 118
   td48 5'-TTCCTGGCAggctagctacaacgaGCTGCCCTC-3'
SEQ ID No: 119
   td49 5'-GTGGACGTAggctagctacaacgaAGGCGGTTT-3'
SEQ ID No: 120
   td50 5'-GCGGGTGGAggctagctacaacgaGTACAGGCG-3'
SEQ ID No: 121
   td51 5'-CCTGGCGCAggctagctacaacgaCCAGTGCGC-3'
SEQ ID No: 122
   td52 5'-CAAATGAAAggctagctacaacgaTTCCTGGCG-3'
SEQ ID No: 123
   td53 5'-TTTCCCAAAggctagctacaacgaGAAACTTCC-3'
SEQ ID No: 124
   td54 5'-ATTGTTGGAggctagctacaacgaGCCCCCTTG-3'
SEQ ID No: 125
   td55 5'-TGGGTCACAggctagctacaacgaTGTTGGACG-3'
SEQ ID No: 126
   td56 5'-TCTGGGTCAggctagctacaacgaATTGTTGGA-3
SEQ ID No: 127
   td57 5'-GCACAATCAggctagctacaacgaCTGGGTCAC-3'
SEQ ID No: 128
   td58 5'-GGAGCACAAggctagctacaacgaCATCTGGGT-3'
SEQ ID No: 129
   td59 5'-ACTGGAGCAggctagctacaacgaAATCATCTG-3'
SEQ ID No: 130
   td60 5'-ATGGAGGGAggctagctacaacgaTGGAGCACA-3'
SEQ ID No: 131
   td61 5'-TGGTACTTAggctagctacaacgaGGAGGGACT-3'
SEQ ID No: 132
   td62 5'-GGGCTGGTAggctagctacaacgaTTATGGAGG-3'
SEQ ID No: 133
   td63 5'-TCAACGATAggctagctacaacgaGCAGCCGGG-3'
SEQ ID No: 134
   td64 5'-CCTCAACGAggctagctacaacgaATGCAGCCG-3'
SEQ ID No: 135
   td65 5'-TCACCTCAAggctagctacaacgaGATATGCAG-3'
SEQ ID No: 136
   td66 5'-CGTCGTTCAggctagctacaacgaCTCAACGAT-3'
SEQ ID No: 137
   td67 5'-GTAAAGATAggctagctacaacgaGCGTGTTGG-3'
SEQ ID No: 138
   td68 5'-AAGTAAAGAggctagctacaacgaATGCGTGTT-3'
SEQ ID No: 139
   td69 5'-GGCAATGAAggctagctacaacgaTGGGTTTCT-3'
SEQ ID No: 140
   td70 5'-TCACGGCAAggctagctacaacgaGAACTGGGT-3'
SEQ ID No: 141
   td71 5'-AGGCAGTCAggctagctacaacgaGGCAATGAA-3'
SEQ ID No: 142
   td72 5'-ATCTGGGCAggctagctacaacgaTCTGGTAGG-3'
SEQ ID No: 143
   td73 5'-GCTGAGTAAggctagctacaacgaCTCGGCATT-3'
SEQ ID No: 144
   td74 5'-TATTATCAAggctagctacaacgaTTTCAGCTG-3'
SEQ ID No: 145
   td75 5'-GGGTTATTAggctagctacaacgaCAATTTTCA-3'
SEQ ID No: 146
   td76 5'-AAGGGGTTAggctagctacaacgaTATCAATTT-3'
SEQ ID No: 147
   td77 5'-CTCCCGGAAggctagctacaacgaCCTTTGGCA-3'
SEQ ID No: 148
   td78 5`-GTACATGGAggctagctacaacgaTCAAAGTTC-3'

Ganz besonders bevorzugt wird als hydrophiler Wirkstoff ein Oligonukleotid mit einer Sequenz SEQ ID No: 40, SEQ ID No: 139 oder SEQ ID No: 140 verwendet.

Darüber hinaus weisen die untersuchten Formulierungen Rez. 1 bis 3 und die Submicroemulsion folgende Zusammensetzung auf (WO = Zusammensetzung der W/O-Primäremulsion; WOW: Zusammensetzung der multiplen Emulsion):

| Rezeptur 1 | | | [Gew-%] |
|---|---|---|---|
| | WO: | Abil EM 90 | 3 |
| WOW_242 | | Mittelkettige Triglyceride | 17 |
| | | MgSo4 * 7H2o | 0.7 |
| | | H2O (konserviert wie untenstehend) | 79.3 |
| | | | |
| | WOW: | WO | 80 |
| | | Poloxamer 407 | 0.8 |
| | | H2O (konserviert wie untenstehend) | 19.2 |
| | | | |
| | Konservierung: | Kaliumsorbat | 0.14 |
| | | Zitronensäure | 0.07 |
| | | | |

| Rezeptur 2 | | | [Gew-%] |
|---|---|---|---|
| | WO: | Sorbitan Monooleate | 10 |
| WOW_167 | | Paraffin dickflüssig | 39.5 |
| | | Lecithin | 0.5 |
| | | NaCl | 0.185 |
| | | H2O (konserviert wie untenstehend) | 49.815 |
| | | | |
| | WOW: | WO | 40 |
| | | Steareth-20 | 1 |
| | | H2O (konserviert wie untenstehend) | 59 |
| | | | |
| | Konservierung: | Parabene | 0.1 |

| Rezeptur 3 | | | [Gew-%] |
|---|---|---|---|
| | WO: | Sorbitan Monooleate | 10 |
| WOW_146 | | Paraffin dickflüssig | 39.5 |
| | | Lecithin | 0.5 |
| | | MgSO4 * 7H2O | 0.77 |
| | | H2O (konserviert wie untenstehend) | 49.23 |
| | | | |
| | WOW: | WO | 40 |
| | | Steareth-20 | |
| | | H2O (konserviert wie untenstehend) | 59 |
| | | | |
| | Konservierung: | Parabene | 0.1 |
| | | | |

| Rezeptur | | | [Gew-%] |
|---|---|---|---|
| | | Coco-caprylate/caprate | 5 |
| Submicronemulsion | | Cetearyl isononanoate | 5 |
| | | oleate | 5 |
| | | Oleth-3 | 0.414 |
| | | Oleth-10 | 5.586 |
| | | Glycerol 85% | 3 |
| | | MgSO₄· 7H₂O | 0.3 |
| | | H2O (konserviert wie untenstehend) | 75.7 |
| | | | |
| | Konservierung: | Kaliumsorbat | 0.14 |
| | | Zitronensäure | 0.07 |

Das in Fig. 1 dargestellte Diagramm zeigt deutlich, dass die multiplen Emulsionen, die das DNAzym in der inneren Wasserphase W₂ enthalten, je nach Zusammensetzung bis zu 70% des Oligonukleotids vor DNAse Abbau schützen (Spalten 2 bis 4). Wird das DNAzym nur als wässrige Lösung mit den DNAsen in Kontakt gebracht, erfolgt ein 100%iger Abbau (Spalte 1).

Anhand der Ergebnisse sieht man, dass ein Schutz nur dann gegeben ist, wenn das DNazym sich in der inneren Wasserphase W₂ einer multiplen Emulsion befindet (Einschluss des Wirkstoffs), wird das DNAzym in die äußere Wasserphase W₁ inkorporiert, so erfolgt, wie im Falle der Submikronemulsion ein 100%iger Abbau (vgl. Spalte 5 und Spalte 6).

Ein Einschluss kann also nur erreicht werden, wenn die Emulsion wie in der allgemeinen Versuchsdurchführung beschrieben, in einem Zweischrittverfahren hergestellt wird. In diesem Fall wird im ersten Schritt eine W/O-Primäremulsion aus der den Komponenten der Ölphase samt dem zugehörigen Emulgator und der wässrigen DNAzym Lösung (W₂) hergestellt. Die so gewonnene W/O-Primäremulsion wird dann im zweiten Schritt mit gegebenenfalls vorhandenen weiteren Hilfsstoffen in eine zweite (DNAzym freie) äußere Wasserphase W₁ gegeben, wobei sich die multiple Wasser-in-Öl-in-Wasser Emulsion ausbildet.

### 5) Scale-Up Versuche

Zur Evaluierung der Scale-Up-Fähigkeit einer erfindungsgemäßen multiplen Emulsion stellt man verschiedene Varianten der Basisemulsion WOW_1 in unterschiedlichen Maßstäben her:

| Formulierung | Maßstab: | Tropfengröße | Viskosität | Phasenstabilität |
|---|---|---|---|---|
| Basisrezeptur WOW_1 Zusammensetzung der Wasserphasen W₁ und W₂ ergänzt um: | 2,5 Kg | +++ | ++ | +++ |
| 0,14% Kaliumsorbat | | | | |
| 0,07% Zitronensäure | | | | |
| Basisrezeptur WOW_1 Zusammensetzung der Wasserphasen W₁ und W₂ ergänzt um: | 12 Kg | +++ | ++ | +++ |
| 0,14% Kaliumsorbat | | | | |
| 0,07% Zitronensäure | | | | |
| | | | | |
| Basisrezeptur WOW_1 ergänzt um: | 2,5 Kg | ++ | ++ | +++ |
| 10% Butylenglycol | | | | |
| Basisrezeptur WOW_1 Zusammensetzung der Wasserphasen W₁ und W₂ ergänzt um: | 2,5 Kg | ++ | ++ | +++ |
| 0,14% Kaliumsorbat | | | | |
| 0,07% Zitronensäure | | | | |
| 3% Glycerin | | | | |
| Vergleichsrezeptur 1: | 2,5 Kg | - | - | - |
| W₂: MgSO4, NaCl Ölphase: Paraffin dickflüssig, Lecithin, Sorbitan Monooleate | | | | (Phasentrennung und/oder Phaseninversion) |
| W₁: Steareth-20 Konservierungsmittel (in den Wasserphasen W₁ und W₂): Benzoesäure | | | | |
| Vergleichsrezeptur 2: | 2,5 Kg | + | ++ | ++ |
| W₂: MgSO4, NaCl Ölphase: Paraffin dickflüssig, Lecithin, Sorbitan Monooleate | | | | |
| W₁: Steareth-20, Sepineo 600 (Acrylamide/ Sodium acryloyldimethyl taurate copolymer/ Isohexadecane/ Polysorbate 80) | | | | |
| Konservierungsmittel (in den Wasserphasen W₁ und W₂): Benzoesäure | | | | |

| | | | | |
|---|---|---|---|---|
| +++ sehr gut ++ gut + befriedigend - schlecht | | | | |

Untersucht man die auf diese Weise hergestellten Emulsionen hinsichtlich ihrer Langzeitstabilität, so ist festzustellen, dass bei denjenigen Emulsionen, die auf der Basisrezeptur WOW_1 basieren, selbst bei Versuchdauern von bis zu 6 Monaten kein Abfall der Stabilität zu beobachten ist. Die untersuchten multiplen Emulsionen auf der Grundlage der Basisrezeptur weisen daher allesamt über eine sehr gute Langzeitstabilität auf.

Die Vergleichsrezeptur 1 liefert überhaupt keine stabile multiple Emulsion; die Vergleichsrezeptur 2 ist nur wenige Wochen stabil.

### 6) Zusatz verschiedener Konservierungsmittel zur Basisrezeptur einer erfindungsgemäßen Emulsion

### 6.1) Einfluss die Stabilität der Emulsion:

Zur Evaluierung der Stabilität einer erfindungsgemäßen Emulsion bei Zusatz verschiedener Konservierungsmittel versetzt man die in Punkt 2) beschriebene Basisrezeptur WOW_1 mit unterschiedlichen Konservierungsmitteln. Hierzu wird das/die jeweilige/n Konservierungsmittel bereits bei der Herstellung der Emulsionen in die beiden wässrigen Phasen W₁ und W₂ gegeben. Anschließend werden die Tropfengröße, die Viskosität und die Phasentrennung untersucht.

Folgende Zusätze können als Konservierungsmittel eingesetzt werden (bei den nachfolgend angeführten Prozentangaben handelt es sich um Gew.-% bezogen auf die Wassermenge): Kaliumsorbat, Zitronensäure, Butylenglycol, Benzoesäure, Propylensäure, Sorbinsäure und Glycerin, nämlich
- 0,14% Kaliumsorbat in Verbindung mit 0,07% Zitronensäure;
- 0,2% Kaliumsorbat in Verbindung mit 0,1% Zitronensäure;
- 10% Butylenglycol;
- 11 % Butylenglycol in Verbindung mit 3% Glycerin;
- 0,05% Benzoesäure;
- 20% Propylenglycol;
- 0,1% Sorbinsäure in Verbindung mit 3% Glycerin; sowie
- 0,12% Sorbinsäure in Verbindung mit 3% Glycerin.

Wie die Versuche zeigen, können verschiedene Konservierungsmittel und -gemische eingesetzt werden, ohne dass die Stabilität der multiplen Emulsion negativ beeinflusst wird. Dies zeigt, dass eine erfindungsgemäße Emulsion unter Zuhilfenahme verschiedener Konservierungsmittel oder -gemische vor mikrobiellem Befall geschützt werden kann, ohne dass die Stabilität der Emulsion beeinträchtigt wird.

Basisrezeptur WOW_1 mit verschiedenen Konservierungsmitteln:

| Zusammensetzung der Emulsion | Tropfengröße | Viskosität | Phasentrennung |
|---|---|---|---|
| Emulsion A: | +++ | +++ | +++ |
| Basisrezeptur | | | |
| WOW_1 | | | |
| Emulsion B: | +++ | ++ | +++ |
| WOW_1 mit | | | |
| 0,14% Kaliumsorbat | | | |
| 0,07% Zitronensäure | | | |
| Emulsion C: | + | + | +++ |
| WOW_1 mit | | | |
| 0,2% Kaliumsorbat | | | |
| 0,1% Zitronensäure | | | |
| Emulsion D: | +++ | ++ | +++ |
| WOW_1 mit | | | |
| 10% Butylenglycol | | | |
| Emulsion E: | ++ | ++ | +++ |
| WOW_1 mit | | | |
| 11% Butylenglycol | | | |
| 3% Glycerin | | | |
| Emulsion F: | +++ | + | +++ |
| WOW_1 mit | | | |
| 0,05% Benzoesäure | | | |
| Emulsioin G: | ++ | ++ | +++ |
| WOW_1 mit | | | |
| 20% Propylenglycol | | | |
| Emulsion H: | ++ | ++ | +++ |
| WOW_1 mit | | | |
| 0,1% Sorbinsäure | | | |
| 3% Glycerin | | | |
| Emulsion I: | ++ | + | +++ |
| WOW_1 mit | | | |
| 0,12% Sorbinsäure | | | |
| 3% Glycerin | | | |

| | | | |
|---|---|---|---|
| Bewertungsschlüssel: +++ sehr gut ++ gut + befriedigend - schlecht | | | |

### 6.2) Mikrobiologische Belastungstests

Zur Evaluierung der Konservierungswirkung kann man, beispielhaft für die in Punkt 7.1) dargestellten multiplen Emulsionen B, C, H und I, einen mikrobiologischen Belastungstest durchführen. Bei dem Belastungstest handelt es sich um ein Standardverfahren, das gemäß dem Europäischen Arzneibuch (Eur. Ph.; Pharmacopoea Europaea) durchgeführt wird. Hierbei erhält man für alle Emulsionen sehr gute Ergebnisse. Auch dies zeigt, dass eine erfindungsgemäße multiple Emulsion durch Zusatz eines Konservierungsmittels wirksam vor mikrobiellem Befall geschützt werden kann.

### 7) Innere Struktur einer erfindungsgemäßen multiplen Emulsion

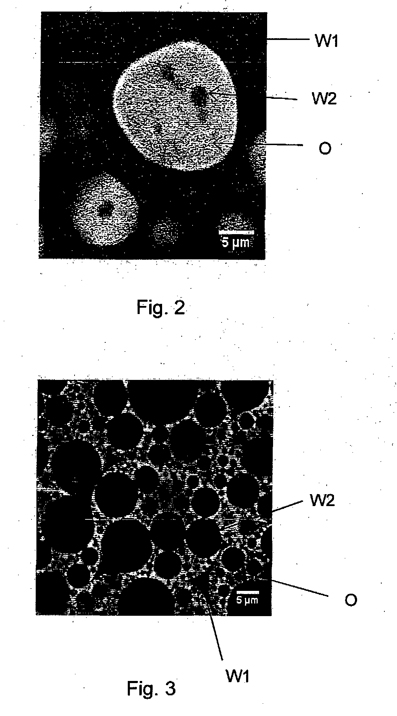

Die in den Fig. 2 und 3 dargestellten Bilder sind mit kohärenter anti-Stokes-Raman-Streuungs-Mikroskopie (engl. Choerent anti-Stokes Raman scattering, CARS) aufgenommen worden. Detektiert wurden symmetrische CH₂ Streckschwingungen bei 2845 cm⁻¹; so dass hierbei die Lipidverteilung abgebildet der jeweiligen Probe abgebildet wird).

Untersucht wurden zum einen, die unter Punkt 2) der Ausführungsbeispiele beschriebene Basisrezeptur WOW_1, und zum anderen eine andersartige multiple Emulsion, die im Unterschied zur erfindungsgemäßen Emulsion Sorbitan Monooleate als innerer, lipophiler Emulgator und Steareth-20 als äußerer, hydrophiler Emulgator, und eine Ölphase aus Paraffin und Lecitihn enthält.

Anhand der CARS Aufnahmen ist ersichtlich, dass die mit der Basisrezeptur WOW_1 gewonnene multiple Emulsion eine wesentlich homogenere innere Struktur mit vielen fein verteilten inneren Wassertropfen zeigt (Fig. 3), als die Vergleichsemulsion mit Sorbitan Monooleate als innerer Emulgator und Steareth-20 als äußerer Emulgator, Paraffin und Lecitihn als Ölphase (Fig. 2). Diese verbesserte innere Struktur der Basisrezeptur, die der erfindungsgemäßen Multiplen Emulsion zu Grunde liegt, scheint für die außerordentliche Stabilität und Schutzfunktion der erfindungsgemäßen Emulsionen verantwortlich zu sein. Dieser Effekt ist im Wesentlichen auf den lipophilen Emulgator Cetyl PEG/PPG-10/1 Dimethicone zurückzuführen. Außerdem scheint die vorteilhafte homogene Struktur dafür verantwortlich zu sein, dass mit Hilfe der erfindungsgemäßen Emulsion große Mengen eines Wirkstoffs, insbesondere große Mengen eines DNAzyms, in der inneren Wasserphase W₂ der Emulsion eingeschlossen werden können.

### 8) DNAzym Wiederfindung

Auf der Grundlage der Zusammensetzung der in Punkt 2) der Ausführungsbeispiele beschriebenen Basisrezeptur WOW_01 können beispielhaft die zwei nachfolgend dargestellten Ausführungsbeispiele V1 und V2 einer erfindungsgemäßen Emulsionen hergestellt werden. Untersucht man nach der Herstellung die in den Emulsionen enthaltene DNAzym-Menge per HPLC untersucht, gelangt man zu folgenden Ergebnissen:
Zusammensetzung der untersuchten Emulsion V1:
   Zusammensetzung der Basisrezeptur WOW_01, sowie zusätzlich
      - in der inneren Wasserphase W₂: 0,4% eines DNAzyms (Na-Salz), Kaliumsorbat* / Zitronensäure** als Konservierungsmittel, und
      - in der äußeren Wasserphase W₁ Kaliumsorbat* / Zitronensäure** als Konservierungsmittel.
         * Menge Kaliumsorbat:14 Gew.-% bezogen auf die Wassermenge
         ** Menge Zitronensäure: 0,07Gew.-% bezogen auf die Wassermenge
   Es konnte eine Wiederfindung für den Wirkstoff (DNAzym) (Wirkstoffmenge mittels HPLC quantifiziert) von 96,67 +/- 2,89% festgestellt werden.
Zusammensetzung der untersuchten Emulsion V2:
   Zusammensetzung der Basisrezeptur WOW_01, sowie zusätzlich
      - in der inneren Wasserphase W₂: 0,4% eines DNAzym (Na-Salz), Kaliumsorbat*/ Zitronensäure**/Glycerin*** als Konservierungsmittel, und
      - in der äußeren Wasserphase W1 Kaliumsorbat*/ Zitronensäure**/Glycerin*** als Konservierungsmittel.
         *Menge Kaliumsorbat:14 Gew.-% bezogen auf die Wassermenge
         ** Menge Zitronensäure: 0,07 Gew.-% bezogen auf die Wassermenge
         ***Menge Glycerin: 3 Gew.-% bezogen auf die Wassermenge

Es konnte eine Wiederfindung für den Wirkstoff (DNAzym) (Wirkstoffmenge mittels HPLC quantifiziert) von 102,00 +/- 6,10% festgestellt werden.

Die Erfindung ist nicht auf eine der vorbeschriebenen Ausführungsformen beschränkt, sondern in vielfältiger Weise abwandelbar.

So ist je nach Anwendungszweck beispielsweise denkbar, dass die erfindungsgemäße multiple Emulsion weitere Bestandteile enthält, die zur Verbesserung der

Hautpflegeeigenschaften, der Konsistenz, der Viskosität und/oder der Penetration des Wirkstoffs vorgesehen sind.

Hierzu zählen - sofern sie nicht als Wirkstoff vorgesehen sind - z.B. Feuchthaltestoffe, Pflegestoffe und Mineralstoffe, wie Glycerin, Urea, Allantoin, Inositol, Zucker, Aminosäuren, Natriumlaktat, wasserlösliche Magnesium- Natrium und/oder Kaliumsalze, die bevorzugt in einem Anteil von 0,1-8 Gew.-% und besonders bevorzugt in einem Anteil von 3 Gew.-% bezogen auf die multiple Emulsion enthalten sind, Vitamine, wie z.B. Retinol, Thiamin, Ascorbinsäure, Calciferol, Riboflavin, Tocopherol, Cobalamin, Pantothensäure, Biotin, Pyridoxin, Niacin und/oder Folsäure, die bevorzugt in einem Anteil von 0,01-5 Gew.-% bezogen auf die multiple Emulsion enthalten sind, pflegenden natürlichen Ölen, wie Nachtkerzen- und/oder Avocadoöl, sowie Paraffinöl zur Okklusion alleine oder in einer Mischung mit mittelkettigen Triglyceriden, Verdicker, wie Carbomere, Cellulose-Derivate, Xanthan, Polymere und/oder Polyethylenglycole.

Stoffe zur Penetrationserhöhung werden bevorzugt in der Ölphase eingesetzt. Hierzu zählen: Lecithin, das bevorzugt in einem Anteil von 0,1-3 Gew.-% bezogen auf die multiple Emulsion enthalten ist, und/oder Fettsäurester der Ölsäure, bevorzugt Decyloleat oder Oleyloleat, bei denen bevorzugt ein Anteil von 0,1-5 Gew.-% bezogen auf die multiple Emulsion vorgesehen ist, ein oder mehrere ein- oder mehrwertige Alkohole, insbesondere Propylenglycol, die bezogen auf die multiple Emulsion bevorzugt in einem Anteil von 0,1-20%, enthalten sind.

Die multiple Emulsion kann neben dem erfindungsgemäß vorgesehenen Wirkstoff weitere Wirkstoffe enthalten. Diese müssen nicht zwingend alle in der inneren Wasserphase W₂ vorliegen, sondern können auch in der Ölphase O oder der äußeren Wasserphase W₁ enthalten sein. So können neben dem erfindungsgemäß vorgesehenen hydrophilen Wirkstoff auch ein mehrere lipophile Wirkstoffe und/oder weitere hydrophile Wirkstoffe enthalten sein.

Sämtliche aus den Ansprüchen, der Beschreibung und der Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Multiple Emulsion zur Applikation wenigstens eines pharmazeutischen oder kosmetischen Wirkstoffs, die eine äußere Wasserphase W₁, eine in der äußeren Wasserphase W₁ dispergierte Ölphase O und eine in der Ölphase O dispergierte innere Wasserphase W₂ aufweist, wobei in der inneren Wasserphase W₂ wenigstens ein Elektrolyt ausgewählt aus der Gruppe der Alkali- und Erdalkalihalogenide und -sulfate und wenigstens ein hydrophiler Wirkstoff vorgesehen ist, **dadurch gekennzeichnet, dass**:
• die äußere Wasserphase W₁ einen hydrophilen Emulgator enthält, der ein Polymer von Ethylenoxid und Propylenoxid ist,
• die Ölphase O von Triacylglyceriden gebildet ist und einen lipophilen Emulgator aus der Gruppe der Dimethicone aufweist und
• der hydrophile Wirkstoff wenigstens ein Oligonukleotid mit einer Sequenz gemäß SEQ ID No: 1 bis SEQ ID No: 148 oder SEQ ID No: 150 ist.

2. Multiple Emulsion nach Anspruch **1, dadurch gekennzeichnet, dass** der hydrophile Emulgator ein Poloxamer ist.

3. Multiple Emulsion nach einem der vorhergehenden Ansprüche, **dadurch gekennzeichnet, dass** der lipophile Emulgator ein lineares Dimethicon mit Polyether- und Alkylgruppen ist.

4. Multiple Emulsion nach einem der vorhergehenden Ansprüche, **dadurch gekennzeichnet, dass** die Triacylglyceride Ester gesättigter Fettsäuren sind.

5. Multiple Emulsion nach einem der vorhergehenden Ansprüche, **dadurch gekennzeichnet, dass** der Elektrolyt NaCl, MgSO₄ oder ein Gemisch aus NaCl und MgSO₄ ist.

6. Multiple Emulsion nach einem der vorhergehenden Ansprüche, **dadurch gekennzeichnet, dass** der hydrophile Wirkstoff MgSO₄ ist.

7. Multiple Emulsion nach einem der vorhergehenden Ansprüche, **dadurch gekennzeichnet, dass** die multiple Emulsion ein oder mehrere Konservierungsmittel enthält.

8. Multiple Emulsion nach einem der vorhergehenden Ansprüche, **dadurch gekennzeichnet, dass** die Topfengröße der dispergierten Ölphase 1 µm bis 100 µm beträgt.

9. Multiple Emulsion nach einem der vorhergehenden Ansprüche, **dadurch gekennzeichnet, dass** in der Ölphase ein gesättigter Kohlenwasserstoff enthalten ist.

10. Multiple Emulsion nach einem der vorhergehenden Ansprüche, **dadurch gekennzeichnet, dass** der hydrophile Emulgator in der äußeren Wasserphase W₁ in einem Anteil von 0,1 bis 5 Gew.-% vorliegt.

11. Multiple Emulsion nach einem der vorhergehenden Ansprüche, **dadurch gekennzeichnet, dass** der lipophile Emulgator bezogen auf eine W/O-Primäremulsion bestehend aus der Ölphase O und der inneren Wasserphase W₂ in einem Anteil von 0,1 bis 6 Gew.-% vorliegt.

12. Multiple Emulsion einem der vorhergehenden Ansprüche, **dadurch gekennzeichnet, dass** die Triglyceride bezogen auf eine W/O-Primäremulsion bestehend aus der Ölphase O und der inneren Wasserphase W₂ in einem Anteil von 5 bis 30 Gew.-% vorliegen.

13. Multiple Emulsion nach einem der vorhergehenden Ansprüche, **dadurch gekennzeichnet, dass** der Elektrolyt bezogen auf eine W/O-Primäremulsion bestehend aus der Ölphase O und der inneren Wasserphase W₂ in einem Anteil von 0,1 bis 3 Gew.-% vorliegt.

14. Multiple Emulsion nach einem der vorhergehenden Ansprüche, **dadurch gekennzeichnet, dass** der hydrophile Wirkstoff bezogen auf die Gesamtmasse der multiplen Emulsion in einem Anteil von 0,01 bis 10 Gew.-% vorliegt.

15. Multiple Emulsion nach einem der vorhergehenden Ansprüche, **dadurch gekennzeichnet, dass**
• der hydrophile Emulgator in der äußeren Wasserphase W₁ einen Anteil von 0,1 bis 5 Gew.-% aufweist, und
• der lipophile Emulgator bezogen auf eine W/O-Primäremulsion bestehend aus der Ölphase O und der inneren Wasserphase W₂ in einem Anteil von 0,1 bis 6 Gew.-% vorliegt, und
• die Triglyceride bezogen auf die W/O-Primäremulsion in einem Anteil von 5 bis 30 Gew.-% vorliegen, und
• der Elektrolyt bezogen auf eine W/O-Primäremulsion bestehend aus der Ölphase O und der inneren Wasserphase W₂ in einem Anteil von 0,1 bis 3 Gew.-% vorliegt, und
• der hydrophile Wirkstoff bezogen auf die Gesamtmasse der multiplen Emulsion in einem Anteil von 0,01 bis 10 Gew.-% vorliegt, und
• in der äußeren Wasserphase W₁ und/oder der inneren Wasserphase W₂ ein Konservierungsmittel in einem Anteil von 0 bis 20 Gew.-% vorgesehen ist.

16. Verwendung einer multiplen Emulsion nach einem der vorhergehenden Ansprüche zur Herstellung eines Arzneimittels, eines Medizinprodukts oder eines Kosmetikums.

17. Verwendung nach Anspruch 16, **dadurch gekennzeichnet, dass** die multiple Emulsion als Wirkstoffträger verwendet wird.

18. Arzneimittel enthaltend eine multiple Emulsion nach einem der Ansprüche 1 bis 15 zur topischen Anwendung oder zur Applikation des Wirkstoffs über Schleimhäute oder zur intraperitonealen Applikation des Wirkstoffs.

19. Arzneimittel enthaltend eine multiple Emulsion nach einem der Ansprüche 1 bis 15 zur Behandlung einer inflammatorischen Erkrankung.

## Claims

1. Multiple emulsion for applying at least one pharmaceutical or cosmetic active substance, which emulsion comprises an external water phase W₁, an oil phase O dispersed in the external water phase W₁ and an internal water phase W₂ dispersed in the oil phase O, at least one electrolyte selected from the group of alkali and alkaline-earth halides and alkali and alkaline-earth sulfates and at least one hydrophilic active substance being provided in the internal water phase W₂, **characterised in that**
• the external water phase W₁ comprises a hydrophilic emulsifier, which is a polymer of ethylene oxide and propylene oxide;
• the oil phase O is formed by triglycerides and comprises a lipophilic emulsifier from the group of dimethicones; and
• the hydrophilic active substance is at least one oligonucleotide having a sequence according to SEQ ID No: 1 to SEQ ID No: 148 or SEQ ID No: 150.

2. Multiple emulsion according to claim 1, **characterised in that** the hydrophilic emulsifier is a poloxamer.

3. Multiple emulsion according to either of the preceding claims, **characterised in that** the lipophilic emulsifier is a linear dimethicone having polyether and alkyl groups.

4. Multiple emulsion according to any of the preceding claims, **characterised in that** the triglycerides are esters of saturated fatty acids.

5. Multiple emulsion according to any of the preceding claims, **characterised in that** the electrolyte is NaCl, MgSO₄ or a mixture of NaCl and MgSO₄.

6. Multiple emulsion according to any of the preceding claims, **characterised in that** the hydrophilic active substance is MgSO₄.

7. Multiple emulsion according to any of the preceding claims, **characterised in that** said multiple emulsion contains one or more preservatives.

8. Multiple emulsion according to any of the preceding claims, **characterised in that** the drop size of the dispersed oil phase is 1 µm to 100 µm.

9. Multiple emulsion according to any of the preceding claims, **characterised in that** a saturated hydrocarbon is contained in the oil phase.

10. Multiple emulsion according to any of the preceding claims, **characterised in that** the hydrophilic emulsifier is present in the external water phase W₁ in a proportion of from 0.1 to 5 wt.%.

11. Multiple emulsion according to any of the preceding claims, **characterised in that** the lipophilic emulsifier is present in a proportion of from 0.1 to 6 wt.%, based on a W/O primary emulsion consisting of the oil phase O and the internal water phase W₂.

12. Multiple emulsion according to any of the preceding claims, **characterised in that** the triglycerides are present in a proportion of from 5 to 30 wt.%, based on a W/O primary emulsion consisting of the oil phase O and the internal water phase W₂.

13. Multiple emulsion according to any of the preceding claims, **characterised in that** the electrolyte is present in a proportion of from 0.1 to 3 wt.%, based on a W/O primary emulsion consisting of the oil phase O and the internal water phase W₂.

14. Multiple emulsion according to claim 1, **characterised in that** the hydrophilic active substance is present in a proportion of from 0.01 to 10 wt.%, based on the total mass of the multiple emulsion.

15. Multiple emulsion according to any of the preceding claims, **characterised in that**
• the proportion of hydrophilic emulsifier in the external water phase W₁ is from 0.1 to 5 wt.%; and
• the lipophilic emulsifier is present in a proportion of from 0.1 to 6 wt.%, based on a W/O primary emulsion consisting of the oil phase O and the internal water phase W₂; and
• the triglycerides are present in a proportion of from 5 to 30 wt.%, based on the W/O primary emulsion; and
• the electrolyte is present in a proportion of from 0.1 to 3 wt.%, based on a W/O primary emulsion consisting of the oil phase O and the internal water phase W₂; and
• the hydrophilic active substance is present in a proportion of from 0.01 to 10 wt.%, based on the total mass of the multiple emulsion; and
• a preservative is provided in the external water phase W₁ and/or in the internal water phase W₂ in a proportion of from 0 to 20 wt.%.

16. Use of a multiple emulsion according to any of the preceding claims for producing a pharmaceutical product, a medicinal product or a cosmetic product.

17. Use according to claim 16, **characterised in that** the multiple emulsion is used as an active-substance carrier.

18. Pharmaceutical product containing a multiple emulsion according to any of claims 1 to 15 for topical use, or for application of the active substance via mucous membranes, or for intraperitoneal application of the active substance.

19. Pharmaceutical product containing a multiple emulsion according to any of claims 1 to 15, for treatment of an inflammatory disease.

## Revendications

1. Emulsion multiple pour l'application au moins d'un principe actif pharmaceutique ou cosmétique, qui présente une phase aqueuse externe W₁, une phase huileuse 0 dispersée dans la phase aqueuse externe W₁ et une phase aqueuse interne W₂ dispersée dans la phase huileuse 0, dans laquelle il est prévu dans la phase aqueuse interne W₂ au moins un électrolyte choisi dans le groupe des halogénures et des sulfates de métaux alcalins et de métaux alcalinoterreux et au moins un principe actif hydrophile, **caractérisée en ce que** :
• la phase aqueuse externe W₁ contient un émulsionnant hydrophile qui est un polymère d'oxyde d'éthylène et d'oxyde de propylène,
• la phase huileuse 0 est formée de triacyl-glycérides et présente un émulsionnant lipophile choisi dans le groupe des diméthicones et
• le principe actif hydrophile est au moins un oligonucléotide présentant une séquence selon la SEQ ID n° 1 à la SEQ ID n° 148 ou la SEQ ID n° 150.

2. Emulsion multiple selon la revendication 1, **caractérisée en ce que** l'émulsionnant hydrophile est un poloxamère.

3. Emulsion multiple selon l'une quelconque des revendications précédentes, **caractérisée en ce que** l'émulsionnant lipophile est un diméthicone linéaire avec des groupements polyéther et alkyle.

4. Emulsion multiple selon l'une quelconque des revendications précédentes, **caractérisée en ce que** les triacylglycérides sont des esters d'acides gras saturés.

5. Emulsion multiple selon l'une quelconque des revendications précédentes, **caractérisée en ce que** l'électrolyte est NaCl, MgSO₄ ou un mélange de NaCl et de MgSO₄.

6. Emulsion multiple selon l'une quelconque des revendications précédentes, **caractérisée en ce que** le principe actif hydrophile est MgSO₄.

7. Emulsion multiple selon l'une quelconque des revendications précédentes, **caractérisée en ce que** l'émulsion multiple contient un ou plusieurs agents de conservation.

8. Emulsion multiple selon l'une quelconque des revendications précédentes, **caractérisée en ce que** la grosseur des gouttes de la phase huileuse dispersée atteint 1 µm à 100 µm.

9. Emulsion multiple selon l'une quelconque des revendications précédentes, **caractérisée en ce qu'**un hydrocarbure saturé est contenu dans la phase huileuse.

10. Emulsion multiple selon l'une quelconque des revendications précédentes, **caractérisée en ce que** l'émulsionnant hydrophile est présent dans la phase aqueuse externe W₁ en proportion de 0,1 à 5 % en poids.

11. Emulsion multiple selon l'une quelconque des revendications précédentes, **caractérisée en ce que** l'émulsionnant lipophile est présente en proportion de 0,1 à 6 % en poids par rapport à une émulsion primaire W/O constituée de la phase huileuse 0 et de la phase aqueuse interne W₂.

12. Emulsion multiple selon l'une quelconque des revendications précédentes, **caractérisée en ce que** les triglycérides sont présents en proportion de 5 à 30 % en poids par rapport à une émulsion primaire W/O constituée de la phase huileuse 0 et de la phase aqueuse interne W₂.

13. Emulsion multiple selon l'une quelconque des revendications précédentes, **caractérisée en ce que** l'électrolyte est présent en proportion de 0,1 à 3 % en poids par rapport à une émulsion primaire W/O constituée de la phase huileuse 0 et de la phase aqueuse interne W₂.

14. Emulsion multiple selon l'une quelconque des revendications précédentes, **caractérisée en ce que** le principe actif hydrophile est présent en proportion de 0,01 à 10 % en poids par rapport à la masse totale de l'émulsion multiple.

15. Emulsion multiple selon l'une quelconque des revendications précédentes, **caractérisée en ce que** :
• l'émulsionnant hydrophile présente dans la phase aqueuse externe W₁ une proportion de 0, 1 à 5 % en poids, et
• l'émulsionnant lipophile est présent en proportion de 0,1 à 6 % en poids par rapport à une émulsion primaire W/O constituée de la phase huileuse 0 et de la phase aqueuse interne W₂, et
• les triglycérides sont présents en proportion de 5 à 30 % en poids par rapport à l'émulsion primaire W/O, et
• l'électrolyte est présent en proportion de 0,1 à 3 % en poids par rapport à une émulsion primaire W/O constituée de la phase huileuse 0 et de la phase aqueuse interne W₂, et
• le principe actif hydrophile est présent en proportion de 0,01 à 10 % en poids par rapport à la masse totale de l'émulsion multiple, et
• un agent de conservation en proportion de 0 à 20 % en poids est prévu dans la phase aqueuse externe W₁ et/ou dans la phase aqueuse interne W₂.

16. Utilisation d'une émulsion multiple selon l'une quelconque des revendications précédentes pour la fabrication d'un médicament, d'un produit médical ou d'un cosmétique.

17. Utilisation selon la revendication 16, **caractérisée en ce que** l'émulsion multiple est utilisée comme support de principe actif.

18. Médicament contenant une émulsion multiple selon l'une quelconque des revendications 1 à 15 pour une application topique ou l'application du principe actif sur des peaux muqueuses ou pour l'application intrapéritonéale du principe actif.

19. Médicament contenant une émulsion multiple selon l'une quelconque des revendications 1 à 15 pour le traitement d'une maladie inflammatoire.
